(19) **Europäisches Patentamt / European Patent Office / Office européen des brevets**

(11) **EP 2 011 433 A1**

(12) # EUROPEAN PATENT APPLICATION

(43) Date of publication:
**07.01.2009 Bulletin 2009/02**

(51) Int Cl.:
*A61B 5/00* (2006.01)     *A61B 5/0488* (2006.01)

(21) Application number: **07120074.5**

(22) Date of filing: **06.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **05.07.2007 US 958334 P**

(71) Applicants:
- **INTERUNIVERSITAIR MICROELEKTRONICA CENTRUM vzw (IMEC)**
  **3001 Leuven (BE)**
- **Universiteit Antwerpen**
  **2000 Antwerpen (BE)**

(72) Inventors:
- **Torfs, Tom**
  **B-3001, Leuven (BE)**
- **Van Hoof, Chris**
  **B-3001, Leuven (BE)**
- **Wuyts, Floris**
  **B-2850, Boom (BE)**
- **Vanspauwen, Robby**
  **B-3740, Bilzen (BE)**

(74) Representative: **Sarlet, Steven Renaat Irène et al**
**Gevers & Vander Haeghen**
**Holidaystraat, 5**
**1831 Diegem (BE)**

(54) **An autonomous wireless system for evoked potential measurements**

(57)   The present invention provides an integrated system for generating a stimulation signal to a person's body and for processing the resulting EMG signals. The system comprises a processing and controlling block having an integrated radio and antenna, an output being arranged for transferring the stimulation signal via an actuator to a person's body and an array of electrodes being attachable to a person's body and being arranged for recording the resulting EMG signal and for transferring this signal to the processing and controlling block. The integrated system wherein the processing and controlling block is arranged for generating a stimulus, for storing and processing the resulting EMG signals, and wherein the processing and controlling block is arranged for sending the processed data via a WL link to a processor.

Fig.2

EP 2 011 433 A1

## Description

### Technical field

The invention relates to the field of evoked potential measurements systems. In particular, an autonomous wireless system is being presented.

### Background art

In evoked potential measurements, an electrical potential is being recorded following presentation of a stimulus, as distinct from spontaneous potentials such as EEG (electroencephalogram) or EMG (electromyogram). A known evoked potential technique, is VEMP, Vestibular Evoked Myogenic Potential.

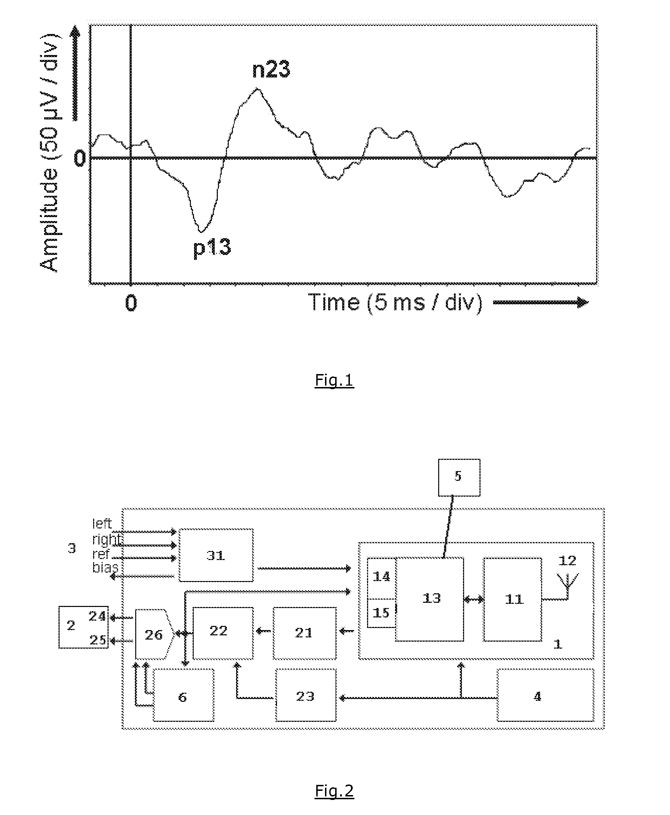

The measurement of VEMPs is a clinical, non-invasive diagnostic method that can identify disorders affecting the balance system. Short, loud tone bursts or clicks are applied to an audio actuator (earphone or bone stimulator), stimulating sensory tissue (otolith organ) in the saccule. Neural impulses travel through the patient's body up to the sternocleidomastoid muscle (anterior muscle in the neck that act to flex and rotate the head). The resulting EMG response is recorded on the sternocleidomastoid muscle synchronously to the applied stimulus. Other variants of VEMP exist where the actuation stimulus is given at a different location and the EMG response is measured on another muscle, but the principle is the same. After averaging a lot of measurements, the response will show positive and negative peaks at approximately 13 and 23 ms, called P13 and N23. A typical plot of the response as a function of time is given in fig. 1. VEMPs may be abnormal (absent, low amplitude, high or enhanced amplitude, or delayed latency) in Meniere's disease, superior canal dehiscence, vestibular neuritis, multiple sclerosis, migraine, spinocerebellar degeneration.

As a specific application, most astronauts experience balance problems during reentry after spaceflight as in "B.J. Yates, I.A. Kerman, 'Post-spaceflight orthostatic intolerance: possible relationship to microgravity-induced plasticity in the vestibular system', Brain Research reviews, Vol.28 (1-2) 1998, pp.73-82.". To study this phenomenon and to determine the proper pharmacological countermeasures, one diagnostic tool is to perform VEMP testing on people. Existing commercial VEMP testing systems are cabled systems, which impede certain experiments, particularly those that involve motion and rotation of the patient.

A wireless VEMP system will facilitate experiments where subjects or patients are subjected to specific movements to enhance stimulation of the balance system. The most straightforward implementation of a wireless system is to use the wireless system as a simple 'wire replacement' and during measurement transmit all acquired data samples wirelessly to a receiver, as in patent EP0852476. All processing and analysis can then be performed at the receiver side. However, such straightforward implementations have several disadvantages. During VEMP measurements, data are steadily acquired at a relatively high sample rate (for example, 5kHz) with audio stimuli repeating at a fixed interval (for example, 5Hz), and this during a relatively long period (30 seconds or more). Therefore a large amount of data (1,000,000 bits per measurement or more) must be transmitted over the radio, leading to a relatively high average power consumption and thus reduced battery autonomy. There would be no time to handle an acknowledge/retransmit scheme (with associated indeterminate transmission delay) in real time, because new data are continuously coming. Since data loss is not acceptable, a large amount of memory (to be used as a FIFO, first input first output, buffer) would be needed to buffer all data, which would also consume additional power.

### Disclosure of the invention

It is an aim of the present invention is to provide an autonomous wireless system for evoked potential measurements.

The present invention provides an integrated system for generating a stimulation signal for a human body part and for processing response signals of the human body part in response to the stimulation signal. The system comprises a processing and controlling block having an integrated radio and antenna for wirelessly transmitting data relating to the response signals to an external device, an output which is arranged for transferring the stimulation signal via an actuator to a person's body, and an array of electrodes which are being attached to a person's body and which are being arranged for recording the response signals and for transferring the response signals to the processing and controlling block. The processing and controlling block is arranged for being attached in the vicinity of the person's body. The processing and controlling block comprises following means: means for locally generating the stimulation signal and means for locally storing and processing the recorded response signals prior to transmittal to the external device. The means for locally generating the stimulation signal as well as the means for locally storing and processing the recorded response signals are preferably formed by appropriate algorithms running on the processing and controlling block.

The integrated system works autonomous. In the prior art, the wireless solutions are simple wire replacements which transmit all acquired data samples during measurement. A processor processes the sampled data and generates a stimulus. The system of the present invention can process, store, retrieve and send response signals e.g. EMG data. In the system of the present invention more intelligence is placed locally in the device which is attached to the person's body. This way the processing of the waveforms is done in the device's digital processor and only the resulting waveforms e.g. VEMP waveforms need to be transmitted (at the end of the measurement and sporadically during the measurement to track the built-up of the waveform). The invention is low-power and reliable, due to the autonomous operation, leading to a strongly reduced amount of data to be transmitted over the wireless link and allowing for handshaking algorithms to implement guaranteed correct transmission and reception of all data over the wireless link.

In a preferred embodiment, the stimulation signal is an audio pulse. To build up the VEMP waveform, the system locally generates the audio impulses (an external trigger input/output is provided to allow generation of the audio impulses by an external device). The system integrates VEMP recording and auditory stimulation. For this preferred embodiment, the actuator can be an earphone or a bone stimulator.

In another embodiment, the stimulation signal may be wirelessly transferred to the actuator. Alternatively, the response signals may also be transferred wirelessly to the processing and controlling block. Avoiding wire connections can enhance the person's comfort.

The integrated system measures the response signals synchronously to the impulse. The processing and controlling block averages the individual resulting measurements. To resolve the evoked potentials against the background of ongoing EEG, ECG or EKG (electrocardiogram), EMG and other biological signals and ambient noise, signal averaging is usually required. In addition, the processing and controlling block further comprises a quality control block. This block locally calculates the mean rectified value of the response signals. It monitors the signal quality and discards measurements below a configurable mean rectified value threshold. By continuously controlling the signal quality, low-quality individual measurements are discarded, improving the quality of the overall resulting waveform after averaging. The integrated system also monitors a maximum signal amplitude threshold level and discards measurements exceeding this threshold level. This way, artifacts in the measurement results (for example, movement artifacts) are avoided. By processing the data locally, less data needs to be transferred over the wireless link, providing a low-power solution needing a limited bandwidth. In the case of VEMP measurements, the user of the system of the present invention will receive processed waveforms. Additionally, the processing and controlling block can look for the specific VEMP peaks in the measurements.

In a preferred embodiment, the system further comprises a feedback tension means, giving an indication of the muscle tension based on said calculated threshold. It provides the signal quality to the patient, allowing him to control his muscle tension. A feedback tension means can use this information and can provide feedback to the patient via e.g. a display. It is difficult for patients to keep their muscles under sufficient tension for a significant amount of time, and from experience it was seen that the real time feedback is very useful, allowing to obtain higher quality VEMP recordings in practical situations. Further, the measurements are faster completed which is positive in view of the patient's comfort.

The parameters under investigation during the VEMP test are the peak latencies and the peak to peak amplitude. In order to obtain a correct interpretation of left-right amplitude differences, it is important to take the sternocleidomastoid muscles (SCM) contraction magnitude into account and to provide the subjects or patients with a visual feedback of this contraction while measuring VEMPs. However, this requires a simultaneous measurement of standard EMG (for the SCM contraction) and averaged evoked potentials (VEMPs), which is not possible in most commercial standard auditory evoked potential devices. The VEMP wireless system is equipped with the necessary amplifiers and software to measure simultaneously the VEMPs and the SCM contraction. Moreover, the possibility to provide the subjects with feedback of their SCM contraction exists also in this system.

In a preferred embodiment, the integrated system further comprises a safety circuit, controlling the stimulation signal. The output sound levels can be very high and could cause damage the patient's hearing if the system were to accidentally give these high sound levels for prolonged periods of time instead of just a short tone burst or click. This safety circuit continuously monitors the average output power level and if it exceeds a set safety threshold, it permanently disconnects the actuators from the system, protecting the patient's ears. Additionally, a measurement in progress can be stopped at any time by the operator. He can monitor the VEMP waveform as it is being built up, and decide to stop the measurement either because it is already sufficiently clear and there is no need to continue averaging, or because he sees there is something wrong (e.g. an EMG electrode is not properly attached).

In a preferred embodiment, an autonomous wireless system for VEMP monitoring is provided comprising the integrated system of the present invention. The innovation in this wireless VEMP system is its applicability in situations where either it is impossible to use the traditional bulky equipment, or where the experimental set-up does not allow the wired connections between subject and recording unit. A direct clinical application is bed-side testing where vertigo patients who can not leave their bed, still can be tested with this system. Additionally, the wireless VEMP is particularly useful when additional loads are imposed on the otolith system. This situation is encountered during rotation experiments where subjects are rotated on a chair with angular rates of 50 to 500 deg/s, in human centrifuges, in space flight, in parabolic flights, or other situations where subjects are subjected to g- forces or g-level transitions.

The disclosed system is preferably used for VEMP monitoring and stimulation.

In another aspect of the invention, a method is presented for generating a stimulation signal for a human body part and for processing response signals with an integrated system as proposed above. The method comprises the steps of: a) generating a stimulation signal in the processing and controlling block and transferring the signal to a person's body via an actuator, b) measuring a plurality of response signals via an array of electrodes and transferring the response signals to the processing and controlling block, c) processing the plurality of response signals whereby a mean value is calculated and d) sending the processed data and/or mean value via a wireless link to an external device.

In an embodiment, the method further comprises the step of performing quality control before sending the processed data to an external device. By continuously controlling the signal quality, low-quality individual measurements are discarded, improving the quality of the overall resulting waveform after averaging. This way, artifacts in the measurement results (for example, movement artifacts) are avoided.

The method can further comprise the step of giving feedback on muscle tension. It provides information on the signal quality to the patient, allowing him to control his muscle tension.

The method can further comprise the step of controlling the amplitude of said stimulation signal by means of a safety circuit. The output sound levels can be very high and could cause damage the patient's hearings if the system were to accidentally give these high sound levels for prolonged periods of time instead of just a short tone burst or click.

### Brief description of the drawings

The invention will be further elucidated by means of the following description and the appended figures.

Fig.1 shows a VEMP measurement result.

Fig.2 shows schematically a wireless VEMP system.

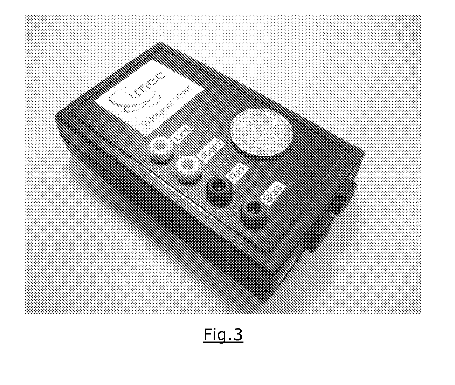

Fig.3 shows a wireless VEMP system prototype.

### Modes for carrying out the invention

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the invention.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting of only components A and B. It means that with respect to the present invention, the only relevant components of the device are A and B.

Disclosed herein is a wireless system which generates an audio stimulus applied to a person's body and which can measure and process the response signals. It will be appreciated, however, that the system embodiments described herein are not limited to wireless VEMP systems as such and can be extended to other wireless systems whereby a stimulus is generated, for example an audio stimulus, an electrical stimulus and whereby the resulting signal is measured and processed, for example, an evoked potential, an EMG signal, or others.

The disclosed invention is in a particular embodiment an integrated system for VEMP monitoring and auditory stimulation. The system integrates VEMP recording and electrical stimulation, all provided by a digital controlling block arranged for controlling the system's functionality meaning: generating the stimulus, receiving the resulting signals and being capable of processing said received data. Further the digital controlling block comprises an integrated antenna for transferring the processed data to a processor via a wireless data link. The integrated system works autonomous. It can process, store, retrieve and send VEMP data.

Fig.1 shows the system of the proposed invention. The system comprises a digital processing block **1** having an integrated radio **11** and antenna **12,** an audio actuator **2** and (EMG) electrodes **3**. The digital block can generate an audio stimulus in its digital signal and processing block **13.** Further the digital signal and processing block **13** has the capability to process and store the incoming EMG signals. The processed data can be sent by a wireless (WL) data link to a processor. The system further comprises amplifiers. There is an amplifier **22** amplifying audio signals coming from the digital-to-analog converter **21.** There is a second amplifier **31** amplifying the incoming EMG signals, which are then converted to digital signals by an ADC **14.** Batteries **4** are also required to provide autonomy to the system.

The system may further comprise two extra blocks. A feedback tension means **5** receives information on the signal quality from the digital signal processing and control block. This information can for example be displayed. Further, a safety circuit **6** can be added, controlling the stimulation signal.

To illustrate the proposed invention, an architecture is presented using specific components. Note that the invention can be implemented using alternative components. The hardware system uses as a core component IMEC's wireless platform, B.Gyselinckx et al. "Human++: Autonomous Wireless Sensors for Body Area networks", Proc. Custom Integrated Circuit Conference (CICC'05) (2005), p.13-19', which is hereby incorporated by reference in its entirety. This platform is combined with a low power microcontroller with 12-bit analog-to-digital converter (ADC) **14** and a low power 2.4GHz radio transceiver **11** and a coplanar integrated antenna **12.**

For the EMG signal amplification, the system uses IMEC's ultra-low power biopotential readout front-end for the EMG signal extraction ('R.F.Yazicioglu et al., "A 60$\mu$W 60nV/$\sqrt{}$Hz Readout Front-End for Portable Biopotential Acquisition Systems", Proceedings of IEEE ISSCC, vol.1 (2006), pp.56-57', which is hereby incorporated by reference in its entirety). It meets the following requirements:

- low voltage battery-powered operation (2.7V-3.3V)
- low power consumption (60 $\mu$W)
- low noise (60nV/$\sqrt{}$/Hz)
- bandwidth (0.1Hz..1850Hz)
- highpass characteristic to reject differential electrode offset
- high common mode rejection ratio (>110 dB even under up to 50 mV DC differential electrode offset)

The output signal is digitized by the 12-bit ADC **14** for further processing.

The audio is synthesized by the digital processor and converted into analog by a 12-bit audio DAC **21** and output filter.

The audio amplifier **22** amplifies the audio signals to drive the actuators. A large dynamic range must be covered, with sound levels from ~0dB nHL (normal hearing level for tone burst/clicks) to ~100 dB nHL. In order to achieve this wide dynamic range, the system does not use a very high resolution audio DAC, which would impose an extremely low noise floor on the system. Instead, a programmable attenuation stage after the amplifier allows attenuating the signal by approx. 45 dB. That way, the dynamic range is split into a strong, unattenuated signal range (> 5mV) and a weak, attenuated signal range (< 5mV).

A fraction of the output signal is fed back through the analog-to-digital converter to the digital processor, allowing the device to self-monitor the exact output signal amplitude.

In order to achieve the highest sound levels with a limited voltage swing, audio actuators with low impedance should be used. Earphones and bone conductors are used with 10 ohms impedance. This allows using a 7.5 to 12V power supply for the audio amplifier. This power supply is created from the nominally 3V battery voltage **4** using a step-up DC/DC converter **23**. The whole audio amplifier block, including the DC/DC converter, is shut down between measurements to conserve battery power.

The output signal can be provided to either the left **24** or the right 25 channel through a dual switch configured as a demultiplexer **26.** Since, unlike in a stereo music player, it is never required to provide different signals simultaneously to both channels, this allows a single audio amplifier for both channels. Since the audio amplifier is one of the most power consuming elements in the system, this improves the battery life.

The peak output sound levels that can be delivered for tone bursts or clicks are very high (for example, 95 dB nHL @ 500Hz corresponds to ~130 dB peak SPL (Sound Pressure Level, referred to the reference pressure $p_0$ = 20$\mu$Pa) for 5-cycle tone bursts. This could lead to hearing damage if the system were to accidentally give these high sound levels for prolonged periods of time instead of just for very brief tone bursts or clicks. Because of this risk, a safety circuit **6** (fully independent from the rest of the controlling electronics) is included, which continuously monitors the average output power level, and if it exceeds a set safety threshold, it permanently disconnects both actuators from the system. The only way to reactivate the actuators in this case is to completely power off and power on the system, thereby also fully resetting the possible fault conditions caused by software or operator errors. If a hardware problem has caused a persistent fault condition, the safety circuit **6** will of course re-activate after the power cycle, continuing to protect the patient.

There are 3 different types of calibration profiles stored in the device's non-volatile memory **15:**

- The device calibration profile contains calibration constants for the specific VEMP device: EMG amplifier gain and audio amplifier gain calibration, for both left and right channels.
- The actuator calibration profiles contain calibration constants for the different audio actuators that will be used with the VEMP device (such as earphones or bone conductor). The calibration constants relate the sound power output in dB SPL (RMS, Root Mean Square) to the drive signal in volts provided to the actuator, and this for the different tone frequencies at which the VEMP device can operate, and for both left and right channels.
- The tone stimulus calibration profiles contain the configuration parameters for a specific tone stimulus (tone bursts/ clicks, tone frequency, duration, plateau/ramp times) combined with the normal hearing level (0dB nHL level) calibration constant for this specific type of tone stimulus.

The device calibration is performed once after the manufacturing of each device:

- The EMG amplifier gain can be automatically calibrated by the device and interface software by applying a stimulus signal of fixed, known amplitude to the EMG inputs from a signal generator.
- The audio amplifier gain can be self-calibrated by the device by measuring the output signal amplitude using a built-in analog-to-digital converter. After this self-calibration, the device can accurately apply desired voltage amplitudes to the actuators.
- The actuator calibration is performed once for each type of actuator used (independent of the device used):

The actuator is driven with pure tone signals of different, known voltage amplitudes.

- A calibration-standard sound power measurement device and coupling device (which can be provided by the actuator manufacturer's distributor) is used to measure the resulting sound output in dB SPL (RMS). It is also possible to use a device that measures the sound output in dB HL (hearing level, for pure tones), the software can take care of the required frequency-dependent translations between dB SPL and dB HL.
- This is repeated at the different tone frequencies at which the VEMP device can operate, and - for two channel actuators - both for left and right channels.
- From the measured curves, the device calculates the frequency-dependent calibration constants which allow to establish the relationship between sound output and applied voltage amplitude:

$$\text{Sound output [dB SPL]}$$
$$= S + 10 \log (P_{el} / 1W)$$
$$= S + 10 \log \{(V / 1V)^2 / (R / 1\Omega)\}$$

$$= C + 20 \log (V / 1V)$$

with:

- $S$ = actuator sensitivity [dB SPL for 1W input]
- $P_{el}$ = electrical power [W]
- $V$ = applied RMS voltage [V]
- $R$ = actuator electrical impedance [ohm]
- $C$ = calibration constant [dB] = $S - 10 \log (R / 1\Omega)$
- After this calibration (and the audio amplifier gain calibration described above), the device, in combination with the specific actuator, can accurately produce desired sound levels in dB SPL.

The tone stimulus calibration is performed once for each tone stimulus used (independent of the device or actuator used):

- The tone stimulus waveform is configured:

    o Click:

      ■ Click duration
      ■ Click polarity

    o Tone burst:

      ■ Tone frequency
      ■ Rise/plateau/fall times
      ■ Window function (linear, Blackman, ...)

The tone stimulus amplitudes are best expressed in dB peak SPL instead of dB SPL (RMS), because the signals are short bursts instead of pure sinusoid tones. Since for the windowed bursts the maximum amplitude is taken equal to the amplitude of the pure tone, the relationship can be described as:

$$\text{Burst level [dB peak SPL]} = \text{pure tone level [dB SPL (RMS)]} + 3dB$$

Subsequently the normal hearing level (OdB nHL level) is determined for the chosen tone stimulus parameters. This level is dependent on the waveform and tone frequency. For example, for 5 cycle tone bursts, the following figures are given in the manual of a commercial audiology system:

| Tone frequency (Hz) | 0dB nHL level in dB peak SPL | Pure tone level in dB SPL (RMS) with amplitude equivalent to the burst peak amplitude |
|---|---|---|
| 250 | 49 | 46 |
| 500 | 35 | 32 |
| 750 | 31 | 28 |
| 1000 | 30 | 27 |

The normal hearing level can be determined by a sufficiently large sample of young, healthy volunteers without hearing damage in an very quiet environment (e.g. soundproof room in a building outside of office hours). To allow this calibration (at levels 90-100 dB below the stimulus levels normally used for measurements), the device features a very large dynamic range for output sound levels.

After this calibration, the device can be used to apply tone burst or click stimulus signals of a well-defined level expressed in dB nHL. All calibration profiles are stored locally in the non-volatile memory of the device. This assures the calibration data stays securely stored in each device, independent of PC software installations.

The device calibration profile is unique for each device. The actuator and tone stimulus calibration profiles will be shared between multiple devices. An automatic programming tool allows to rapidly program these calibration profiles, once established, into all manufactured devices. With the provided interface software, the end user can perform additional calibration if he desires to use a different actuator or a substantially different tone stimulus waveform from the pre-calibrated ones.

During operation, a combination of one of each of these 3 calibration profiles is used:

- The device calibration profile is automatically used by the device.
- The actuator calibration profile is selected by the user depending on which actuator is used; one of the actuator calibration profiles is marked as default and will be used by default (this should correspond to the actuator the user most commonly employs).
- The tone stimulus calibration profile is selected by the user and automatically determines the tone stimulus waveform parameters. To change the parameters from the pre-calibrated profiles, the user will have to modify (a copy of) the tone stimulus calibration profile, and if the changes are substantial he should perform a new normal hearing level calibration or look up the normal hearing level for his chosen waveform in literature. One of the tone stimulus calibration profiles is marked as default and will be used by default (this should correspond to the tone stimulus waveform the user most commonly employs).

**Claims**

1. An integrated system (1) for generating a stimulation signal for a human body part and for processing response signals, said system comprising:

   a processing and controlling block (13) having an integrated radio (11) and antenna (12) for wireless transmitting data relating to said response signals to an external device,
   an output (2) being arranged for transferring said stimulation signal via an actuator to a person's body,
   an array of electrodes (3) being attachable to a person's body and being arranged for recording said response signals and for transferring said response signals to said processing and controlling block (13);

**characterised in that** said processing and controlling block (13) comprises
means for locally generating said stimulation signal;
means for locally storing and processing said recorded response signals prior to transmittal to said external device.

2. An integrated system as in claim 1, **characterised in that** said stimulation signal is an audio pulse.

3. An integrated system as in claim 2, **characterised in that** said actuator is an earphone.

4. An integrated system as in any one of claims 1 and 2, **characterised in that** said actuator is a bone stimulator.

5. An integrated system as in any one of the previous claims **characterised in that** said stimulation signal is wirelessly transferred to said actuator.

6. An integrated system as in any one of the previous claims **characterised in that** said processing and controlling block comprises a quality control block, said quality control block being arranged for calculating a minimum signal quality threshold.

7. An integrated system as in any one of the previous claims **characterised in that** said system further comprising a feedback tension means (5), giving an indication of the muscle tension based on said calculated threshold.

8. An integrated system as in any one of the previous claims **characterised in that** said system further comprising a safety circuit (6), controlling said stimulation signal.

9. An autonomous wireless system for Vestibular Evoked Myogenic Potential monitoring comprising the integrated system of the previous claims.

10. A method for generating a stimulation signal for a human body part and for processing response signals with an integrated system according to any of the previous claims, said method comprising the steps of:

    a) generating a stimulation signal in said processing and controlling block, said stimulation signal being transferred to a person's body via an actuator;
    b) measuring a plurality of response signals via an array of electrodes and transferring said response signals to said processing and controlling block;
    c) processing said plurality of response signals whereby a mean value is calculated;
    d) sending said processed data comprising said mean value via a wireless link to an external device.

11. A method as in claim 10, further comprising the step of performing quality control before sending to an external device.

12. A method as in any of claims 10 and 11, further comprising the step of giving feedback on muscle tension.

13. A method as in any of claims 10 to 12, further comprising the step of controlling the amplitude of said stimulation signal.

Fig.1

Fig.2

Fig.3

European Patent Office

**EUROPEAN SEARCH REPORT**

Application Number

EP 07 12 0074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/047667 A (SARNOFF CORP [US]; FISCHER RUSSELL J [US]; FERRARO JOSEPH [US]; LAL PR) 26 April 2007 (2007-04-26) | 1-4,7-13 | INV. A61B5/00 A61B5/0488 |
| Y | * abstract * * paragraph [0030] - paragraph [0063] * * figures 1,11,12 * | 5,6 | |
| Y | WO 2006/122349 A (COMPUMEDICS MEDICAL INNOVATION [AU]; BURTON DAVID [AU]; ZILBERG EUGENE) 23 November 2006 (2006-11-23) * abstract * * page 12, line 9 - page 25, line 31 * | 5,6 | |
| A | * figures 11,13,15 * | 1-4,7-13 | |
| Y | WO 2005/072459 A (EVEREST BIOMEDICAL INSTR [US]; CAUSEVIC ELVIR [US]) 11 August 2005 (2005-08-11) * abstract * * page 8, paragraph 1 - page 12, paragraph 2 * | 5 | |
| A | * figure 5 * | 1-4,6-13 | TECHNICAL FIELDS SEARCHED (IPC) A61B |
| D,A | WO 97/10747 A (CLEVELAND MED DEVICES INC [US]) 27 March 1997 (1997-03-27) * the whole document * | 1-13 | |

The present search report has been drawn up for all claims

| Place of search | Date of completion of the search | Examiner |
|---|---|---|
| Munich | 16 June 2008 | Artikis, T |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 12 0074

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2008

| Patent document cited in search report | | Publication date | Patent family member(s) | | Publication date |
|---|---|---|---|---|---|
| WO 2007047667 | A | 26-04-2007 | NONE | | |
| WO 2006122349 | A | 23-11-2006 | NONE | | |
| WO 2005072459 | A | 11-08-2005 | US 2007167857 A1 | | 19-07-2007 |
| WO 9710747 | A | 27-03-1997 | AU 7369196 A | | 09-04-1997 |
| | | | CN 1199329 A | | 18-11-1998 |
| | | | DE 69634486 D1 | | 21-04-2005 |
| | | | DE 69634486 T2 | | 05-01-2006 |
| | | | EP 0852476 A1 | | 15-07-1998 |
| | | | JP 11511367 T | | 05-10-1999 |
| | | | US 5755230 A | | 26-05-1998 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0852476 A

**Non-patent literature cited in the description**

- **B.J. YATES ; I.A. KERMAN.** Post-spaceflight orthostatic intolerance: possible relationship to microgravity-induced plasticity in the vestibular system. *Brain Research reviews,* 1988, vol. 28 (1-2), 73-82
- **B.GYSELINCKX et al.** Human++: Autonomous Wireless Sensors for Body Area networks. *Proc. Custom Integrated Circuit Conference (CICC'05,* 2005, 13-19
- **R.F.YAZICIOGLU et al.** A 60μW 60nV/√Hz Readout Front-End for Portable Biopotential Acquisition Systems. *Proceedings of IEEE ISSCC,* 2006, vol. 1, 56-57